# EUROPÄISCHE PATENTSCHRIFT

(11) **EP 1 418 921 B1**
(45) Veröffentlichungstag und Bekanntmachung des Hinweises auf die Patenterteilung: **03.10.2007**
(21) Anmeldenummer: 02762422.0
(22) Anmeldetag: 01.08.2002
(51) Int. Cl.: A61K 31/565, C07J 31/00, A61P 35/00

(54) **ANTITUMOR WIRKSAME 2-ALKOXYESTRADIOLSULFAMATE**
ANTI-TUMOUR 2-ALKOXYESTRADIOL SULFAMATES
SULFAMATES DE 2-ALKOXYESTRADIOL PRESENTANT UNE ACTION ANTITUMORALE

(30) Priorität: 13.08.2001 DE 10139494
(43) Veröffentlichungstag der Anmeldung: 19.05.2004
(73) Patentinhaber: Sterix Limited, Slough, Berkshire SL1 3XE (GB)
(72) Erfinder: SCHERLITZ-HOFMANN, Ina, 14612 Falkensee (DE); HOFFMANN, Jens, 16567 Mühlenbeck (DE); HILLISCH, Alexander, 07743 Jena (DE); UNGER, Eberhard, 07751 Cospeda (DE); NEUMANN, Tobias, 07743 Jena (DE); SCHWARZ, Sigfrid, 07743 Jena (DE); PETERS, Olaf, 99891 Tabarz (DE); MICHEL, Thomas, 04157 Leipzig (DE)
(74) Vertreter: Bradley, Adrian
(86) Internationale Anmeldenummer: PCT/EP2002/008597
(87) Internationale Veröffentlichungsnummer: WO 2003/015792

(56) Entgegenhaltungen:
- WO-A-93/05064
- WO-A-96/05217
- US-A- 6 136 992
- MACCARTHY-MORROGH L ET AL: "DIFFERENTIAL EFFECTS OF ESTRONE AND ESTRONE-3-O-SULFAMATE DERIVATIVES ON MITOTIC ARREST, APOPTOSIS AND MICROTUBULE ASSEMBLY IN HUMAN BREAST CANCER CELLS" CANCER RESEARCH, AMERICAN ASSOCIATION FOR CANCER RESEARCH, BALTIMORE, MD, US, Bd. 60, Nr. 19, 1. Oktober 2000 (2000-10-01), Seiten 5441-5450, XP001031282 ISSN: 0008-5472 in der Anmeldung erwähnt
- CUSHMAN M ET AL: "SYNTHESIS, ANTITUBULIN AND ANTIMITOTIC ACTIVITY, AND CYTOTOXICITY OF ANALOGS OF 2-METHOXYESTRADIOL, AN ENDOGENOUS MAMMALIAN METABOLITE OF ESTRADIOL THAT INHIBITS TUBULIN POLYMERIZATION BY BINDING TO THE COLCHICINE BINDING SITE" JOURNAL OF MEDICINAL CHEMISTRY, AMERICAN CHEMICAL SOCIETY. WASHINGTON, US, Bd. 38, Nr. 12, 1995, Seiten 2041-2049, XP002055798 ISSN: 0022-2623

## Beschreibung

Die vorliegende Erfindung betrifft 2-Alkoxyestradiolsulfamate und deren Verwendung zur Herstellung von Arzneimitteln, die eine Antitumor wirksame Aktivität aufweisen.

Mikrotubuli sind Organellen, die in den meisten eukaryontischen Zellen vorkommen und dort eine Reihe von. Funktionen wie Mitose, intrazelluläre Bewegungen, Zellwanderung und die Ausprägung der Zellform übernehmen. Mikrotubuli sind Polymere aus Tubulin, das seinerseits ein Dimer aus einer α- und einer β-Einheit darstellt. Diese Heterodimere binden zwei Moleküle Guanosintriphosphat (GTP), wobei eines der GTPs fest gebunden und das andere austauschbar ist. Die Heterodimere polymerisieren in einer Kopf-Schwanz-Anordnung zu fadenförmigen Makromolekülen, den sogenannten Protofilamenten, die sich ihrerseits zu röhrenförmigen Organellen, den Mikrotubuli, zusammenlagern. Mikrotubuli unterliegen einem ständigen Auf- und Abbau. Das Gleichgewicht zwischen Wachstum und Abbau hängt von der Verfügbarkeit neuer GTP-Tubulin-Untereinheiten und der Hydrolysegeschwindigkeit des zweiten gebundenen GTPs ab. Am Plus-Ende werden neue Untereinheiten angebaut, wogegen am Minus-Ende Untereinheiten abdiffundieren. Es ist bekannt, dass zytotoxische Substanzen wie Colchicin, Vinblastin, Vincristin, Taxol, Epothilone, Podophyllotoxin, Steganicin, Combretastatin, Methoxyestradiol den Auf- bzw. Abbau der Mikrotubuli (Tubulinpolymerisation und Tubulindepolymerisation) beeinflussen und somit in der Lage sind, die Zellteilung phasenspezifisch zu beeinflussen. Dies betrifft vor allem schnell wachsende, neoplastische Zellen, deren Wachstum durch intrazelluläre Regelmechanismen weitgehend unbeeinfilusst ist. Wirkstoffe dieser Art sind prinzipiell geeignet zur Behandlung maligner Tumoren.
Fotsis et. al. Nature 1994.368, 237-239 berichten darüber, dass 2-Methoxyestradiol das Tumorwachstum und die Angiogenese hemmt.
Cushman et. al. J. Med. Chem. 1995 38, 2041-2049 untersuchen die zytotoxische sowie die Tubulin-Polymerisationshemmende Wirkung von 2-Methoxyestradiol, und berichten in J. Med Chem. 1997, 40, 2323-2334 darüber, dass 2-Alkoxy-6-oximinoestradiol-Derivate die Tubulinpolymerisation sowie die Bindung von [³H]-Cholchicin an Tubulin hemmen. Die hier genannten 2-Alkoxy-6-oximinoestradiol-Derivate zeigen bzgl. der Hemmung der. Tubulinpolymerisation vergleichbare Aktivität wie 2-Ethoxyestradiol, das eine höhere Aktivität als 2-Methoxyestradiol aufweist.

In der Bestrebung, die orale Bioverfügbarkeit von 2-Alkoxyestradiol, insbesondere von 2-Methoxyestradiol zu steigern, wurde gemäß vorliegender Erfindung die 3-Hydroxyfunktion ausgewählter 2-Alkoxyestradiolderivate sulfamoyliert. Dabei konnte festgestellt werden, dass die resultierenden 2-Alkoxyestradiolsulfamate nicht nur besser bioverfügbar sind, sondern die Tubulinpolymerisation überraschenderweise stärker hemmen als die 2-Alkoxyestradiole selbst. Folglich sind die entsprechenden erfindungsgemäßen Sulfamate auch *in vivo* antitumor wirksam.

Speziell für Estradiol und nahe verwandte Derivate war bereits bekannt, dass die Einführung einer Sulfamatgruppe in vivo zu einer erhöhten oralen Bioverfügbarkeit führt (Elger et. al. J. Steroid Biochem. and Mol. Biol. 1995, 55, 395). Die verbesserte Bioverfügbarkeit resultiert aus der Bindung dieser Substanzen an Erythrozyten, in denen sie langsamer metabolisiert und sukzessive freigesetzt werden. Die nicht sulfamoylierten Estrogene unterliegen dagegen bei oraler Applikation einer sehr schnellen hepatischen Metabolisierung (first pass effect). Die Synthese von Estrogensulfamaten wird von Schwarz et. al. Steroids 1996, 61, 710-717 beschrieben.

Steroid-3-sulfamate werden andererseits in der Literatur als Hemmstoffe der Steroidsulfatase beschrieben:
WO93/05064 betrifft u.a. Verbindungen der Formel 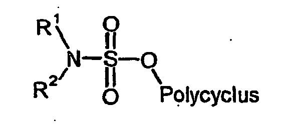 wobei R¹ und R² jeweils unabhängig Wasserstoff oder Methylgruppe bedeuten, mit der Maßgabe, dass mindestens einer der Reste R¹ und R² ein H-Atom ist, und der Rest-O-Polycyclus ein 3-Sterol ist, dessen Sulfatester durch ein Enzym mit Steroidsulfatase-Aktivität hydrolisierbar ist. In der 2-Stellung des Steroidgerüstes spezifisch substituierte Verbindungen sind nicht explizit offenbart.
US 6,011,024 beruht auf der WO93/05064 und deckt z. B. alle Verbindungen ab, in denen die primäre Sulfamatfunktion an einem Sechsring gebunden ist. In der 2-Stellung des Steroidgerüstes spezifisch substituierte Verbindungen sind wiederum nicht explizit offenbart.
WO 96/05216 betrifft an C2-unsubstituierte Estra-1,3,5(10)-trien-Sulfamat-Derivate.
WO 96/05217 betrifft pharmazeutische Zusammensetzungen enthaltend Wirkstoffe der allgemeinen Formel 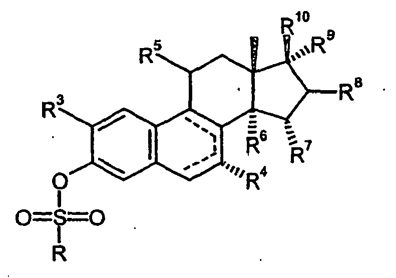 worin R = NH₂; R³ = C₁₋₅-Alkoxygruppe, OH; R⁸, R⁹ und R¹⁰ voneinander unabhängig = H, OH; R⁹ und R¹⁰ zusammen = O die Bedeutung haben können. Die darin offenbarten pharmazeutischen Zusammensetzungen können zur weiblichen Fertilitätskontrolle; klimakterischen HRT und zur Behandlung gynäkologischer und andrologischer Krankheitsbilder, wie Mamma-, bzw. Prostatakarzinom verwendet werden.
WO 97/14712 betrifft Steroidsutfamat-Derivate der allgemeinen Formel 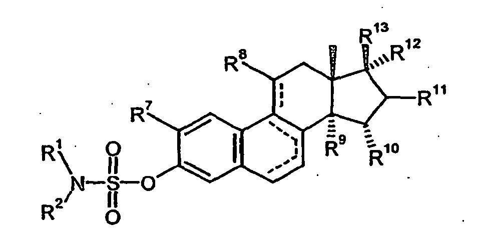 worin R¹ eine Acyl-, Alkoxycarbonyl-, Aminocarbonyl-, Sulfonyl- oder Sulfonamidylgruppe; R² ein Wasserstoff- oder ein Metallatom; R⁷ und R⁸ unabhängig voneinander H, OH und C₁₋₅-Alkoxy; R¹³, R¹², R¹¹ unabhängig voneinander H oder OH darstellen können.
WO 98/42729 betrifft 16-Halogensubstituierte-1,3,5-estratriene-3-monosulfamate sowie 3,17β-Bissulfamate, die an C2 alkoxysubstituiert sein können. Die 16-Halogensubstitution erhöht sowohl die Sulfatase-Hemmwirkung als auch die Estrogenität der entsprechenden Sulfamat-Derivate. Die Einführung einer zu der 3-Sulfamat- zusätzlichen 17-Sutfamatfunktion setzt die Estrogenität drastisch herab.
WO 98/24802 betrifft Sulfamate, die die Estronsulfatase hemmen. 2-Methoxyestronsulfamat wird explizit genannt. Als potentielles Therapiegebiet findet Mammakarzinom, nicht jedoch Prostatakarzinom in der Beschreibung Erwähnung. Auch WO 99/33858 beschreibt Estronsulfatase-Inhibitoren der Formel 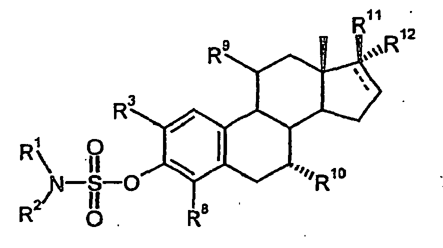 worin R¹ und R² unabhängig voneinander H, Alkyl, oder zusammen Piperidin-, Morpholin, Piperazin; R³ = H, CN, NO₂, CO₂R⁴; R⁸ = H, NO₂, NR⁶R⁷ darstellen. In der Beschreibung ist als mögliches Therapiegebiet Mammakarzinom erwähnt.
WO 99/64013 betrifft eine pharmazeutische Zusammensetzung eines Sulfamat-Derivates mit einem Zellsignalmodifier (wie z.B. TNFα). 2-Methoxyestronsulfamat wird in dieser Kombination als bevorzugtes Sulfamat explizit beansprucht; es fallen aber zahlreiche weitere Steroid-3-sulfamate unter den Umfang der allgemeinen Formel. Als Wirkmechanismus für die erfindungsgemäßen pharmazeutischen Zusammensetzungen bzw. für die darin enthaltenen Steroid-3-sulfamate (bevorzugt mit mindestens einem 2-Alkoxysubstituenten) werden 1) Hemmung der Glucoseaufnahme in Tumorzellen, 2) Hemmung der Tumorangiogenese, 3) Abbau der Mikrotubuli; 4) Induzierung von Apoptose beschrieben. WO 00/76487 betrifft Stoffe, die die TNFα-induzierte Aromatase-Aktivität hemmen. Als solche werden 2-Alkcoxyestron-3-sulfamate, bevorzugt 2-Methoxyestronsulfamat- beansprucht.
WO 01/18028 beschreibt nicht-estrogene Estronsulfatase-inhibierende *N*-Acyl-18a-substituierte Steroid-3-sulfamate, wie z.B. 16α-Fluor-2-methoxy-18α-homoestradiol-(*N-*acetylsulfamat) bzw. 16α-Fluor-2-methoxy-18a-homoestron-(*N*-acetylsuffamat).

In Cancer 2000, 85, 983-994 werden die 2-Methoxyestradiol-, Docetaxel- und Paclitaxel-induzierte Apoptose in Hepatomazellen und deren Korrelation mit reaktiven Sauerstoffspezien verglichen.
Potter et. al. lnt. J. Cancer 2000, 85, 584-589 untersuchen die Wirkung von 2-Methoxyestronsulfamat im Vergleich zu 2-Methoxyestron auf das Wachstum von Brustkrebszellen und induzierte Mammatumoren und finden, dass 2-Methoxyestronsulfamat ein beachtliches therapeutisches Potential zur Behandlung von Brustkrebs aufweist.
Potter et. al. Molecular and Cellular Endocrinology 2000, 160, 61-66 untersuchen die Hemmung der Deoxyglucoseaufnahme in MCF-7-Brustkrebszellen durch 2-Methoxyestron und 2-Methoxyestron-3-sulfamat, die die Glucoseaufnahme um 25 bis 49% bei 10µM (ebenso 2-Methoxyestradiol und 2-Methoxyestron) hemmen, und folgern, dass die Verbindungen durch ihr Vermögen die Glucoseaufnahme zu hemmen, therapeutisches Potential zur Hemmung von Brustkrebs haben könnten.
Potter et. al. Cancer Research 2000, 60, 5441-5450 beschreibt 2-Methoxyestronsulfamat und 2-Ethoxyestronsulfamat als neue antimikrotubullenaktive Verbindungen, die *in vitro* Antikrebsaktivität in Mammakarzinomzellen aufwiesen, und daher auch eventuell *in vivo* aktiv sein könnten. In J. Steroid Biochem. and Mol. Biol. 1999, 69, 227-238 wird berichtet, dass die Hemmung der Steroidsutfätase-Aktivität ein wichtiger Ansatzpunkt bei der Behandlung von hormonabhängigen Mammakarzinomen ist. Explizit werden 2-Methoxyestronsulfamat, 17-Deoxyestronsulfamat und Estronsulfamat aufgeführt. Mono- bzw. bicyclische, nicht steroidale Sulfamate hemmen zwar die Steroidsulfatase, jedoch nicht so effektiv wie die entsprechenden Steroidderivate.

Die Aufgabe der vorliegenden Erfindung besteht darin, Verbindungen zur Verfügung zu stellen, die die Tubulinpolymerisation stärker hemmen als 2-Methoxyestradiol und gleichzeitig eine höhere orale Bioverfügbarkeit aufweisen.

Die Aufgabe der vorliegenden Erfindung wird daher erfindungsgemäß durch die Bereitstellung von Verbindungen der allgemeinen Formel I zur Herstellung eines Arzneimittels zur Behandlung von Tumorerkrankungen, die sich durch die Hemmung der Tubulinpolymerisation positiv beeinflussen lassen, gelöst: 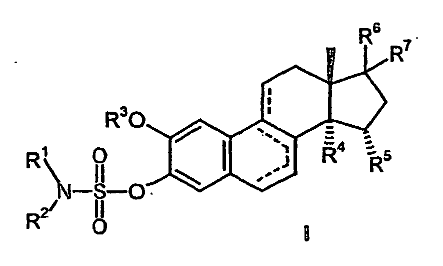 worin
- R¹ und R²: unabhängig voneinander H, Methyl, C₁-C₄-Acyl, Benzoyl,
- R³: C₁-C₄-Alkyl oder einen Rest der Formel CₙFₘHₒ, wobei n = 1, 2, 3, 4, 5 oder 6, m > 1 und m + o = 2n + 1 ist, bedeutet,
- R⁴ und R⁵: jeweils H oder zusammen eine Methylengruppe bzw. eine zusätzliche Doppelbindung,
- R⁶: H,
- R⁷: OH, OC₁-C₄-Alkyl, OC₁-C₁₁-Acyl oder OSO₂NR¹R² darstellen,
wobei im B- und C-Ring des Steroidgerüstes die gestrichelten Linien zusätzlich bis zu zwei Doppelbindungen sein können, mit der Maßgabe, dass die Verbindung etwas anderes ist als

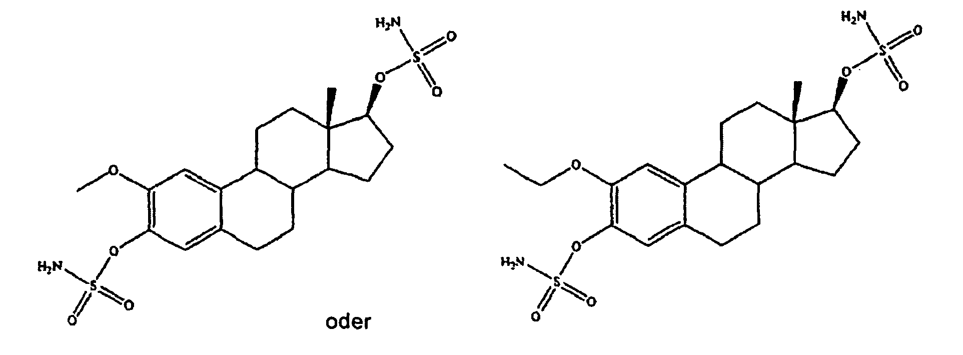

Es wurde festgestellt, dass die erfindungsgemäßen 2-Alkoxyestradiolsulfamate *in vitro* die Tubulinpotymerisation überraschend stärker hemmen als 2-Methoxyestradiol selbst Die erfindungsgemäßen Verbindungen zeigen auch *in vivo* Antitumorwirkung.
Die verbesserte Tubulin-Polyrnerisationshemmung lässt eine stärkere Hemmung des Tumonrvachstums erwarten.

Bei den C₁-C₄-Alkylgnippen für R³ kann es sich durchweg um eine Methyl-, Ethyl-, n-Propyl-, iso-Propyt-, n-, iso-, oder tert.-Butylgruppe handeln. Eine Methyl- oder Ethylgruppe ist bevorzugt.

Für einen Acylrest R¹ und R² kann ein Formyl-, Acetyl-, Propionyl-, Butyryl- oder iso-Butyrylrest stehen.

Für den teilweise oder vollständig fluorierten Alkylrest im Substituenten R³ kommt beispielsweise ein Trifluormethyl-, Pentafluorethyl- oder 2,2,2-Trifluorethylrest in Frage.

Für den Alkoxyrest R⁷ kann eine Methoxy-, Ethoxy-, n-Propoxy-, iso-Prooxy-, n-, iso-, oder tert.-Butoxygruppe stehen.

Eine Acyloxygruppe R⁷ ist beispielsweise eine Acetyloxy-, Propionyloxy-, Butyryioxyiso-Butyryloxy-, Valeryloxy-, Decyloxy- oder Undecyloxygruppe.

Bevorzugt gemäß vorliegender Erfindung ist die Verwendung solcher Verbindungen der allgemeinen Formel I, in denen:
- R¹: H, Methyl, Acetyl, Propionyl, Butyryl
- R²: H
- R³: C₁-C₄-Alkyl, oder CH₂CF₃
- R⁴ und R⁵: jeweils H oder zusammen eine Methylengruppe
- R⁶: H,
- R⁷: OH, OC₁-C₄-Alkyl, OC₁-C₁₁-Acyl oder OSO₂NR¹R² darstellen,
wobei im B- und C-Ring des Steroidgerüstes die gestrichelten Linien zusätzlich eine 8-Doppelbindung bedeuten kann.

Die nachstehend genannten Verbindungen sowie deren Verwendung sind erfindungsgemäß besonders bevorzugt:
1) 2-Methoxy-17β-hydroxy-estra-1,3,5(10)-trien-3-yl-sulfamat
2) 2-Methoxy-17α-hydroxy-estra-1,3,5(10)-trien-3-yl-suffamat
3) 2-Methoxy-17β-hydroxy-estra-1,3,5(10)-trien-3-yl-*N*-acetylsulfamat
4) 2-Methoxy-17α-hydroxy-estra-1,3,5(10)-trien-3-yl-*N*-acetylsulfamat
5) 2-Methoxy-17β-hydroxy-14α,15α-methylen-estra-1,3,5(10)-trien-3-yl-sulfamat
6) 2-Methoxy-17α-hydroxy-14α,15α-methylen-estra-1,3,5(10)-trien-3-yl-sulfamat
7) 2-Methoxy-17β-hydroxy-14α,15α-methylen-estra-1,3,5(10)-trien-3-yl-*N*-acetylsulfamat
8) 2-Methoxy-17α-hydroxy-14α,15α-methylen-estra-1,3,5(10)-trien-3-yl-*N*-acetylsulfamat
9) 2-Methoxy-17β-hydroxy-14α,15α-methylen-estra-1,3,5(10),8-tetraen-3-yl-sulfamat
10) 2-Methoxy-17α-hydroxy-14α,15α-methylen-estra-1,3,5(10),8-tetraen-3-yl-sulfamat
11) 2-Methoxy-17β-hydroxy-14α,15α-methylen-estra-1,3,5(10),8-tetraen-3-yl-*N-*acetylsulfamat
12) 2-Methoxy-17α-hydroxy-14α,15α-methylen-estra-1,3,5(10),8-tetraen-3-yl-*N-*acetylsulfamat
13) 2-Ethoxy-17β-hydroxy-estra-1,3,5(10)-trien-3-yl-sulfamat
14) 2-Ethoxy-17α-hydroxy-estra-1,3,5(10)-trien-3-yl-sulfamat
15) 2-Ethoxy-17β-hydroxy-estra-1,3,5(10)-trien-3-yl-*N*-acetylsulfamat
16) 2-Ethoxy-17α-hydroxy-estra-1,3,5(10)-trien-3-yl-*N*-acetylsulfamat
17) 2-Ethoxy-17β-hydroxy-14α,15α-methylen-estra-1,3,5(10)-trien-3-yl-sulfamat
18) 2-Ethoxy-17α-hydroxy-14α,15α-methylen-estra-1,3,5(10)-trien-3-yl-sulfamat
19) 2-Ethoxy-17β-hydroxy-14α,15α-methylen-estra-1,3,5(10)-trien-3-yl-*N*-acetylsulfamat
20) 2-Ethoxy-17α-hydroxy-14α,15α-methylen-estra-1,3,5(10)-trien-3-yl-*N*-acetylsulfamat
21) 2-Ethoxy-17β-hydroxy-14α,15α-methylen-estra-1,3,5(10),8-tetraen-3-y-sulfamat
22) 2-Ethoxy-17α-hydroxy-14α,15α-methylen-estra-1,3,5(10),8-tetraen-3-yl-sulfamat
23) 2-Ethoxy-17β-hydroxy-14α,15α-methylen-estra-1,3,5(10),8-tetraen-3-yl-*N-*acetylsulfamat
24) 2-Ethoxy-17α-hydroxy-14α,15α-methylen-estra-1,3,5(10),8-tetraen-3-yl-*N-*acetylsulfamat
25) 2-(2',2',2'-Triftuorethoxy)-17β-hydroxy-estra-1,3,5(10)-trien-3-yl-sulfamat
26) 2-(2',2',2'-Trifluorethoxy)-17α-hydroxy-estra-1,3,5(10)-trien-3-yl-sulfamat
27) 2-(2',2',2'-Trifluorethoxy)-17β-hydroxy-estra-1,3,5(10)-trien-3-yl-*N*-acetylsulfamat
28) 2-(2',2',2'-Trifluorethoxy)-17α-hydroxy-estra-1,3,5(10)-tnen-3-yl-M-acetylsulfamat
29) 2-(2',2',2'-Trifluorethoxy)-17β-hydroxy-14α,15α-methylen-estra-1,3,5(10)-trien-3-yl-sulfamat
30) 2-(2',2',2'-Trifluorethoxy)-17α-hydroxy-14α,15α-methylen-estra-1,3,5(10)-trien-3-yl-sulfamat
31) 2-(2',2',2'-Trifluorethoxy)-17β-hydroxy-14α,15α-methylen-estra-1,3,5(10),8-tetraen-3-yl-sulfamat
32) 2-(2',2',2'-Trifluorethoxy)-17α-hydroxy-14α,15α-methylen-estra-1,3,5(10),8-tetraen-3-yl-sulfamat
33) 2-(2',2',2'-Trifluorethoxy)-17β-hydroxy-14α,15α-methylen-estra-1,3,5(10)-trien-3-yl-*N*-acetylsulfamat
34) 2-(2',2',2'-Trifluorethoxy)-17α-hydroxy-14α,15α-methylen-estra-1,3,5(10)-trien-3-yl-*N*-acetylsulfamat
35) 2-(2',2',2'-Trifluorethoxy)-17β-hydroxy-14α,15α-methylen-estra-1,3,5(10),8-tetraen-3-yl-*N*-acetylsulfamat
36) 2-(2',2',2'-Trifluorethoxy)-17α-hydroxy-14α,15α-methylen-estra-1,3,5(10),8-tetraen-3-yl-*N*-acetylsulfamat

### Pharmakologische Daten

Die erfindungsgemäßen Verbindungen wurden in verschiedenen Modellen getestet.
Die erfindungsgemäßen Verbindungen der allgemeinen Formel I zeichnen sich dadurch aus, dass sie die Tubulinpolymerisation stärker hemmen als 2-Methoxyestradiol. Die *in vitro* Testung der Tubulinpolymerisationsbeeinflussung wurde folgendermaßen durchgeführt:
Mikrotubuläres Protein wurde nach Shelanski et. al. (Shelanski et. al. Proc. Natl. Acad. Sci. USA 1973, 70, 765-8) über zyklische Assemblierung / Deassemblierung aus Schweinehirn gereinigt. Das verwendete Buffersystem hatte folgende Zusammensetzung: 20 mM PIPES (1,4-Piperazine-diethane-sulfonsäure, pKa 6,8), 80 mM NaCl, 0,5 mM MgCl₂, 1 mM EGTA (Ethylenglykol-bis-(2-aminoethyle)-tetraessigsäure).
Für die Wirkstofftestung wurden Proteinkonzentrationen von 1 mg/ml (ca. 10⁻⁵ mM Tubulin) eingesetzt. Die Proteinbestimmung erfolgte nach der Lowry-Methode (Lowry et al. J. Biol. Chem. 1951, 193, 265-75) mit Rinderserumalbumin als Standard. Die Assemblierung von Mikrotubuli erfolgte in Gegenwart von 0.25 mM GTP und Erwärmen der Proben auf 37 °C.
Die Mikrotubulusbildung wurde mit Hilfe der Turbidimetrie bei einer Wellenlänge von 340 nm geprüft. Der Gleichgewichtszustand, bei dem das mikrotubuläre Protein keinen Zuwachs der Assemblatkonzentration (entsprechend der Mikrotubuluskonzentration) und der Trübungswert keinen Anstieg mehr aufweist, wird typischerweise nach 20 Minuten erreicht.
Die Testung der Wirkstoffe erfolgte durch deren Zugabe am Anfang der Assemblierung oder im Gleichgewichtszustand. Abweichungen der Trübungskurven von der Kontrolle charakterisieren ihre Wirksamkeit. Zur Wirkungskontrolle und Bewertung der Trübungsmesswerte wurde stets eine Transmissionselektronenmikroskopische Untersuchung (CEM 902 A, Zeiss / Oberkochen) der Assemblate nach Negativfärbung mit 1 %igem wässrigen Uranylazetat ausgeführt.

**Tab. 1.**

| **Name** | **Struktur** | **Hemmung der Tubulinpolymerisation IC₅₀ [µM]** |
|---|---|---|
| 2-Methoxyestradiol | 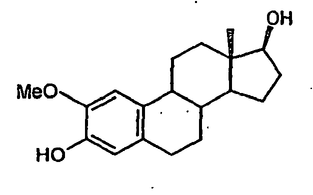 | >3 |
| 2-Methoxyestron-3-sulfamat | 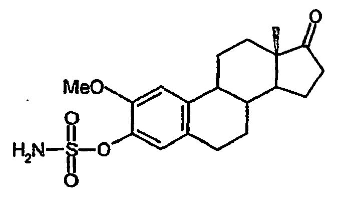 | 1,6 |
| 2-Methoxy-17β-estradiol-3-sulfamat | 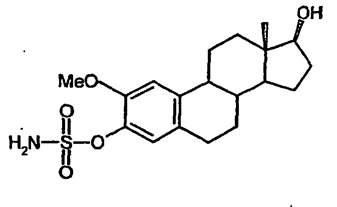 | 1,0 |

In den hier aufgeführten Schemata wurden die folgenden Abkürzungen benutzt:
- Casodex:: *N*-[4'-Cyan-3-(trifluorphenyl]-3-[(4-fluorphenyl)sulfonyl]-2-hydroxy-2-methylpropanarnid
- A:: 2-Methoxy-17β-estradiol-3-sulfamat (Beispiel 1);
- B:: 2-Methoxy-17β-estradio-3-(*N*-acetylsuffamat) (Beispiel 6);

In der vorliegenden Erfindung wurde die Wirkung von den erfindungsgemäßen 2-Alkoxyestradiolsulfamaten auf das Tumorwachstum *in vivo* mittels Maus-Xenograft-Modellen untersucht, bei denen die erfindungsgemäßen Verbindungen kontinuierlich subkutan verabreicht wurden.
Das PC82 Tumormodell (G. Steenbruegge, University of Rotterdam, The Netherlands Literatur Steenbrugge et a. J. Urology 131:812-817, 1984) ist ein hormonabhängiges humanes Prostatakarzinom Modell. Das Tumormodell wurde durch "sertat passaging" von Prostatakarzinomgewebe, das während einer OP entnommen wurde, auf immundefizienten nude Mäusen etabliert und weiter propagiert. Das androgenabhängige LNCaP Prostatakarzinom Modell wurde ebenfalls von einem Patiententumor etabliert. Dieses Tumormodell wächst sowohl in Zellkultur als auch als Xenotransplantat auf immundefizienten Mäusen (Culig, Hoffmann Brit. J Cancer, 1999, 242-251). Für Therapieversuche werden 6 Wochen alte männliche Nacktmäuse, (NMRI-Maus, M&B, Bomholdtgard, Denmark) mit Testosteron-Pellets (12.5 mg, 90 Tage Freisetzung; IRA, Sarasota, FL) supplementiert. Den Tieren werden entweder LNCaP Zellen (1.5 x 10⁶ Zellen) oder kleine PC82 Tumorfragmente (2 x 2 mm) implantiert (subkutan in die linke Flanke). Nachdem die Tumoren eine Größe von 20-25 mm² erreicht hatten, wurde die Behandlung mit den Erfindungssubstanzen begonnen.

Die Ergebnisse sind in Schema 1 und Schema 2 dargestellt. Während in den unbehandelten Kontrolltieren der Tumor schnell wächst führt die Behandlung mit den Erfindungssubstanzen zu einer deutlichen Wachstumshemmung der Prostatatumoren. Im PC82 Modell ist diese Wachstumshemmung vergleichbar mit den Effekten eines Antiandrogens bzw. der Kastration.
Im LNCaP Modell ist ebenfalls eine dosisabhängige Hemmung des Tumorwachstums zu erkennen. Der Effekt ist auch hier vergleichbar der Kastration und sogar überlegen der Behandlung mit dem Antiandrogen Casodex.
Die Ergebnisse der Versuche in den Prostatakarzinom Modellen zeigen eine Hemmung des Tumorwachstums durch die erfindungsgemäßen Verbindungen.

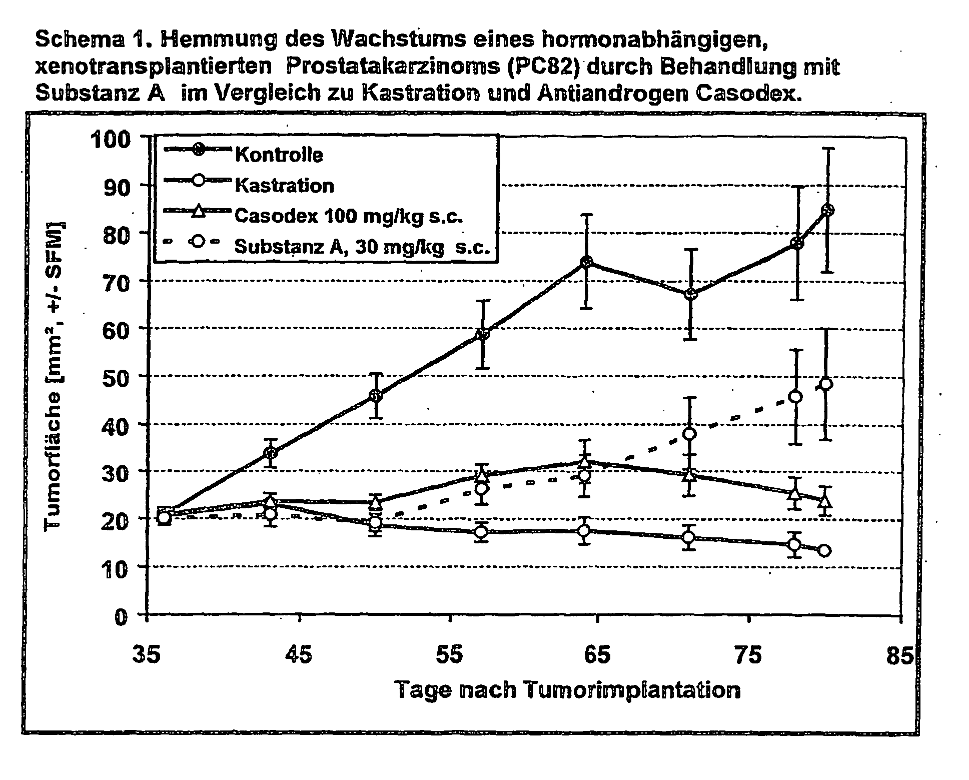

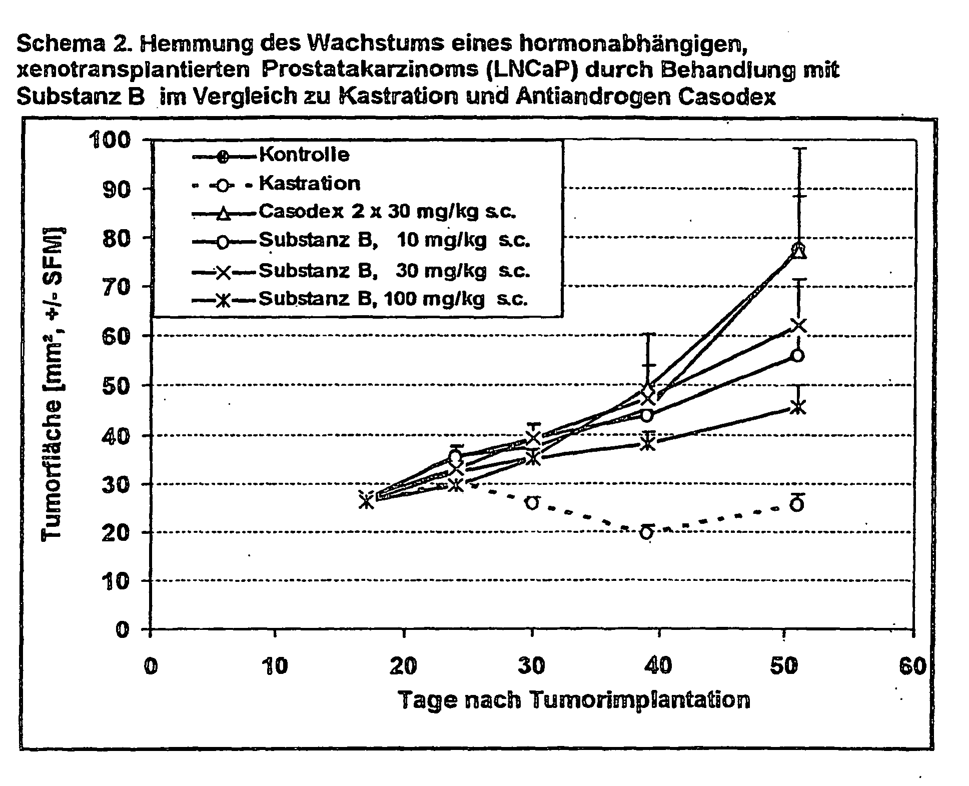

In der vorliegenden Erfindung wurde die Wirkung von den erfindungsgemäßen 2-Alkoxyestradiolsulfamaten auf das Tumorwachstum *in vivo* mittels eines Maus-Xenograft-Modells untersucht, bei dem die erfindungsgemäßen Verbindungen kontinuierlich subkutan verabreicht wurden. Im Vergleich zu den unbehandelten Kontrolltieren resultierte eine Hemmung des Tumorwachstums. Retardation des Tumorwachstums zeigte sich als signifikant in kastrierten Mäusen. Die Behandlung wurde gut vertragen. Im Gegensatz zum Kastrationsmodell wurde kein Schrumpfen der Mausprostata und der Samenblase beobachtet.
Die vorliegende Erfindung zeigt, dass die erfindungsgemäßen Verbindungen eine Inhibition des Prostatatumorvvachstums verursachen.

### Dosierung

Im allgemeinen sind zufriedenstellende Resultate zu erwarten, wenn die täglichen Dosen einen Bereich von 5 µg bis 50 mg der erfindungsgemäßen Verbindung-pro kg Körpergewicht umfassen. Bei größeren Säugetieren, beispielsweise dem Menschen, liegt eine empfohlene tägliche Dosis im Bereich von 10 µg bis 30 mg pro kg Körpergewicht.

Geeignete Dosierungen für die erfindungsgemäßen Verbindungen betragen von 0,005 bis 50 mg pro Tag pro kg Körpergewicht, je nach Alter und Konstitution des Patienten, wobei die notwendige Tagesdosis durch Einmal- oder Mlehrfachabgabe appliziert werden kann.
Aufgrund der besonderen Depotwirkung der Estrogen-Sulfamate können die erfindungsgemäßen Verbindungen aber auch in größeren Abständen als einmal am Tag verabreicht werden **(**WO 00/06175**).**

Die Formulierung der pharmazeutischen Präparate auf Basis der neuen Verbindungen erfolgt in an sich bekannter Weise, indem man den Wirkstoff mit den in der Galenik gebräuchlichen Trägersubstanzen, Füllstoffen, Zerfallsbeeinflussern, Bindemitteln, Feuchthaltemitteln, Gleitmitteln, Absorptionsmitteln, Verdünnungsmitteln, Geschmackskorrigentien, Färbemitteln usw. verarbeitet und in die gewünschte Applikationsform überführt. Dabei ist auf Remington's Pharmaceutical Science, 15th ed. Mack Publishing Company, East Pennsylvania (1980) hinzuweisen.

Für die orale Applikation kommen insbesondere Tabletten, Dragees, Kapseln, Pillen, Pulver, Granulate, Pastillen, Suspensionen, Emulsionen oder Lösungen in Frage.
Für die parenterale Applikation sind Injektion- und Infusionszubereitungen möglich.
Für die intraartikulären Injektion können entsprechend zubereitete Kristallsuspensionen verwendet werden.
Für die intramuskuläre Injektion können wässrige und ölige Injektionslösungen oder Suspensionen und entsprechende Depotpräparationen Verwendung finden.
Für die rektale Applikation können die neuen Verbindungen in Form von Suppositorien, Kapseln, Lösungen (z.B. in Form von Klysmen) und Salben sowohl zur systemischen als auch zur lokalen Therapie verwendet werden.

Zur pulmonalen Applikation der neuen Verbindungen können diese in Form von Aerosolen und Inhalaten verwendet werden.
Für die topische Auftragung sind Formulierungen in Gele, .Salben, Fettsalben, Cremes, Pasten, Puder, Milch und Tinkturen möglich. Die Dosierung der Verbindungen der allgemeinen Formel I sollte in diesen Zubereitungen 0,01% - 20% betragen um eine ausreichende pharmakologische Wirkung zu erzielen.

Die vorliegende Erfindung umfasst die Verwendung der erfindungsgemäßen Verbindungen der allgemeinen Formel I zur Herstellung eines Arzneimittels zur Behandlung von Tumorerkrankungen, die sich durch die Hemmung der Tubulinpolymerisation positiv beeinflussen lassen.
Die erfindungsgemäßen Verbindungen der allgemeinen Formel I werden bevorzugt verwendet zur Herstellung eines Arzneimittels zur Behandlung von Tumorerkrankungen der männlichen und weiblichen Keimdrüsen, männlichen und weiblichen Sexualorgane einschließlich der Brustdrüsen, insbesondere von Prostatakarzinomen oder Mammakarzinoma.

Ferner betrifft die vorliegende Erfindung pharmazeutische Zusammensetzungen, die mindestens eine erfindungsgemäß besonders bevorzugte Verbindung, gegebenenfalls in Form eines pharmazeutisch/pharmakologisch verträglichen Salzes, ohne oder zusammen mit pharmazeutisch verträglichen Hilfs- und/oder Trägerstoffen enthalten.
Diese pharmazeutischen Zusammensetzungen und Arzneimittel können zur oralen, rektalen, vaginalen, subkutanen, perkutanen, intravenösen oder intramuskulären Applikation vorgesehen sein. Sie enthalten neben üblichen Träger- und/oder Verdünnungsmitteln mindestens eine erfindungsgemäß besonders bevorzugte Verbindung.
Die Arzneimittel der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder auch Depotformen.

Die pharmazeutischen Zusammensetzungen enthaltend mindestens eine der erfindungsgemäßen Verbindungen werden bevorzugt oral appliziert.

Es kommen auch parenterale Zubereitungen wie Injektionslösungen in Betracht. Weiterhin seien als Zubereitungen beispielsweise auch Suppositorien und Mittel zur vaginalen Anwendung genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees, durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Verbindungen der allgemeinen Formel I können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten.

Die Verbindungen der allgemeinen Formel I enthaltende Kapseln können beispielsweise hergestellt werden, indem man die Verbindung(en) der allgemeinen Formel I mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyethylenglykol bzw. deren Derivaten herstellen.

Die erfindungsgemäßen Verbindungen können zur Therapie von Prostatakarzinomen mit einem oder mehreren der folgenden Wirkstoffen kombiniert verabreicht werden:
1) Antiandrogene wie CPA, Flutamid, Casodex etc.
2) Gonadrotrophormon (GnRH) Agonisten
3) 5α-Reduktase Hemmer wie Finasterid
4) Zytostatika
5) VEGF-Kinase-Inhibitoren
6) Antigestagene
7) Antiestrogene
8) Antisense Oligonukleotide
9) EGF-Antikörper
10) Estrogene

Darüber hinaus können die erfindungsgemäßen Verbindungen der allgemeinen Formel I zur Therapie und Prophylaxe weiterer oben nicht genannter Krankheitszustände eingesetzt werden.

Die erfindungsgemäßen Verbindungen der allgemeinen Formel I lassen sich wie nachstehend beschrieben herstellen:

Die elektrophile Substitution des C-Atoms 2 eines Estra-1,3,5(19)-trien-17-on-derivates, beispielsweise Verbindungen 1-3, erfolgt vorzugsweise durch Friedel-Crafts-Acylierung wie in der Literatur beschrieben (T. Nambara, S. Honma, S. Akiyama, Chemical and Pharmaceutical Bulletin 1979, 18, 474-480). Nach dem Wechsel der Schutzgruppen in Position 3 wird durch Baeyer-Villiger-Oxidation ein 2-Carboxy-estra-1,3,5(10)-trien-17-on generiert (March, Advanced Organic Chemistry, 3. Edition, J. Wiley & Sons 1985, 990-991 und hierin zitierte Literatur). Der Ester wird verseift und mit einem entsprechenden Alkylhalogenid unter Verwendung einer Base in einen 2-Alkylether überführt. Die Spaltung der Schutzgruppe in Position 3 erfolgt wie in der Literatur beschrieben (T. W. Greene, P. G. M. Wuts, Protective Groups in Organic Synthesis, J. Wiley & Sons, 1999, S.249-275).
Die 2-Acylierung, ausgehend von Verbindungen der allgemeinen Formel II, bei denen R² und R³ zusammen eine Methylenbrücke bilden bzw. die zusätzlichen Doppelbindungen im Steroidgerüst besitzen, wird durch *ortho*-Metallierung realisiert (D. J. Pert, D. D. Ridley, Australian Journal of Chemistry 1989, 42, 405-419; V. Snieckus, Chemical Reviews 1990, 90, 879-933; P. Beak, R. A. Brown, Journal of Organic Chemistry, 1982, 47, 34-46). 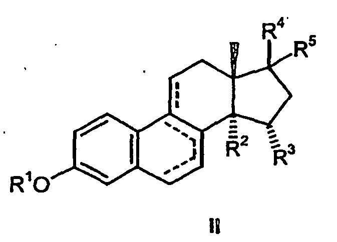 Für R¹ als *ortho*-dirigierende Schutzgruppe in Position 3 wird vorzugsweise eine Carbamatgruppe verwendet. Die elektrophile Substitution erfolgt nach 2-Lithiierung mit Dimethylformamid. Das 2-Formyl-estradiol-derivat wird zum Ameisensäureester oxidiert und in bekannter Weise in eine 2-Alkoxyverbindung umgewandelt **(**WO 98/40398**).** Die Herstellung der Verbindungen der allgemeinen Formel II, in denen R² und R³ zusammen eine Methylenbrücke bilden bzw. die zusätzlichen Doppelbindungen im Steroidgerüst besitzen, gehört zum Stand der Technik (z.B. DE 4239945**;** DE 4239946**;** R. Prousa, B. Schönecker, D. Tresselt, K. Ponsold, Journal für Praktische Chemie 1986, 328, 55-70; H.-J. Siemann, P. Droescher, B. Undeutsch, S. Schwarz, Steroids 1995, 60, 308-315).

Die vorliegende Erfindung wird anhand der nachfolgenden Beispiele näher erläutert, ohne sie darauf einzuschränken:

### Herstellungsverfahren

### 1) Allgemeine Vorschrift zur Herstellung von Estra-1,3,5(10)-trien-3yl -sulfamaten bzw. Estra-1,3,5(10),8-tetraen-3yl-sulfamaten

Es werden 7 mmol eines Estra-1,3,5(10)-trien-17-on-Derivates in 100 ml Methylenchlorid unter Rühren suspendiert und 35 mmol 2,6-Di-*tert*.-butylpyridin sowie 70 mmol Sulfamoylchlorid bei Raumtemperatur zugegeben. Es entsteht eine weiße bis gelbliche Suspension, die 2h weitergerührt wird. Der Ansatz wird mit Wasser versetzt, mit Ethylacetat extrahiert und die Extrakte mit Wasser und gesättigter Natriumhydrogen-carbonatlösung neutral gewaschen. Die organische Phase wird über Natriumsulfat getrocknet, das Lösungsmittel abdestilliert und das Rohprodukt isoliert. Nach Chromatographie an Kieselgel mit einem Eluenten aus Toluol/Ethylacetat (v/v = 10/1) wird das Produkt in Ausbeuten zwischen 70 und 85 % isoliert.

### 2) Allgemeine Vorschrift zur Acetylierung von Estra-1,3,5(10)-trien-3yl-sulfamaten bzw. Estra-1,3,5(10),8-tetraeca-3yl-sarlfamaten

10,5 mmol eines wie beschrieben hergestellten Steroidsulfamates werden in 50 ml Pyridin gelöst und bei einer Temperatur von 0 bis 5°C mit 115 mmol Essigsäureanhydrid versetzt. Es entsteht eine klare Lösung, die 1h bei Raumtemperatur weitergerührt wird. Der Ansatz wird auf Eis gegossen und mit Ethylacetat extrahiert. Nach Ansäuern der Extrakte mit 6N Salzsäure wird mit Wasser und Natriumchloridlösung gewaschen. Die organische Phase wird getrocknet und das Lösungsmittel abdestilliert. Das getrocknete Rohprodukt wird ohne Reinigung für weitere Reaktionen verwendet.

### 3) Allgemeine Vorschrift zur Reduktion der 3-Sulfamate sowie der N-Acetylsulfamate

12 mmol Steroid werden in 120 ml eines Gemischs aus THF/Methanol (1:1) gelöst und bei Raumtemperatur portionsweise mit 100 mmol Natriumborhydrid versetzt. Die Reaktionslösung wird 1h bei Raumtemperatur gerührt und anschließend nach Zugabe von Wasser mit 1M Salzsäure angesäuert. Nach Extraktion mit Ethylacetat wird die organische Phase mit Natriumchloridlösung neutral gewaschen, getrocknet und das Lösungsmittel abdestilliert. Nach Reinigen des Rohproduktes an Kieselgel mit Totuot/Ethytacetat (v/v = 3/1) erhält man das Reduktionsprodukt in guten bis sehr guten Ausbeuten (60-95 % d. Th.).

Folgende erfindungsgemäßen Verbindungen wurden nach den genannten Vorschriften hergestellt:

### Beispiel 1

### 2-Methoxy-17β-hydroxy-estra-1,3,5(10)-trien-3-yl-sulfamat 1:

Nach allgemeiner Synthesevorschrift 1 und 3 wurden aus 13,98 mmol 2-Methoxy-17-oxo-estra-1,3,5(10)-trien-3-ol 10,44 mmol **1** (75 % d. Th.) erhalten.
¹H-NMR (400 MHz, DMSO-d₆, TMS) δ (ppm): 7,80 (s, 2H, -NH₂), 6,97 (s, 1H, H-1), 6,96 (s, 1H, H-4), 4,52 (d, J = 4,7 Hz, 1H, 17β-OH), 3,76 (s, 3H, 2-OCH₃). 3,58-3,49 (m, 1H, H-17α), 2,77-2,68 (m, 2H, H-6), 0,68 (s, 3H, H-18).

### Beispiel 2

### 2-Methoxy-17β-hydroxy-14α,15α-methylen-estra-1,3,5(10)-trien-3-yl-sulfamat 2:

Nach allgemeiner Synthesevorschrift 1 und anschließender Abspaltung der *tert*-Butyldimethylsilyl-Schutzgruppe (T. W. Greene, P. G. M. Wuts, Protective Groups in Organic Synthesis, J. Wiley & Sons, 1999, S.273-276) wurden aus 2,33 mmol 17β-*tert*-Butyldimethylsilyloxy-2-methoxy-14α,15α-methylen-estra-1,3,5(10)-trien-3-ol 1,42 mmol **2** (61 % d. Th.) erhalten.
¹H-NMR (400 MHz, DMSO-d₆, TMS) δ (ppm): 7,80 (s, 2H, -NH₂), 6,95 (s, 1H, H-1), 6,94 (s, 1 H, H-4), 5,67 (s, 1 H, 17β-OH), 3,74 (s, 3H, 2-OCH₃), 3,56-3,50 (m, 1H, H-17α), 2,80-2,62 (m, 2H, H-6), 0,89 (s, 1H, H-18), 0,28-0,20 (m, 2H, 14α,15α-CH₂).

### Beispiel 3

### 2-Ethoxy-17β-hydroxy-estra-1,3,5(10)-trien-3-yl-sulfamat 3:

Nach allgemeiner Synthesevorschrift 1 und 3 wurden aus 1,64 mmol 2-Ethoxy-17oxo-estra-1,3,5(10)-trien-3-ol 1,23 mmol **3** (75 % d. Th.) erhalten.
¹H-NMR (400 MHz, DMSO-d₆, TMS) δ (ppm): 7,76 (s, 2H, -NH₂), 6,96 (s, 1H, H-1), 6,95 (s, 1H, H-4), 4,53 (d, J = 3,9 Hz, 1H, 17β-OH), 4,07-3,99 (m, 2H, 2-OCH₂CH₃, 3,59-3,48 (m, 1H, H-17α), 2,76-2,69 (m, 2H, H-6), 1,31 (t, J = 7,0 Hz, 3H, 2-OCH₂CH₃), 0,71 (s, 3H, H-18).

### Beispiel 4

### 2-Methoxy-17β-hydroxy-estra-1,3,5(10)-trien-3-yl-N-acetylsulfamat 4:

Nach allgemeiner Synthesevorschrift 1, 2 und 3 wurden aus 1,43 mmol 2-Methoxy-17-oxo-estra-1,3,5(10)-trien-3-ol 1,05 mmol **6** (73 % d. Th.) erhalten.
¹H-NMR (400 MHz, DMSO-d₆, TMS) δ (ppm): 12,20 (s, 1H, -NH), 6, 97 (s, 1 H, H-1), 6,92 (s, 1 H, H-4), 4,51 (d, J = 3,5 Hz, 1 H, 17β-OH). 3,73 (s, 3H, 2-OCH₃), 3,57-3,48 (m, 1 H, H-17α), 2,75-2,67 (m, 2H, H-6), 1,99 (s, 3H, -NCOCH₃), 0,68 (s, 3H, H-18).

### Beispiel 5

### 2-Methoxy-17β-hydroxy-14α,15α-methylen-estra-1,3,5(10)-trien-3-yl-N-acetylsulfamat 5:

Nach allgemeiner Synthesevorschrift 1 und 2 sowie anschließender Abspaltung der *tert-*Butyldimethylsilyl-Schutzgruppe (T. W. Greene, P. G. M. Wuts, Protective Groups in Organic Synthesis, J. Wiley & Sons, 1999, S.273-276) wurden aus 2,33 mmol 17β-*tert-*Butyldimethylsilyloxy-2-methoxy-14α,15α-methylen-estra-1,3,5(10)-trien-3-ol 1,19 mmol **7** (51% d. Th.) erhalten.
¹H-NMR (400 MHz, DMSO-d₆, TMS) δ (ppm): 12,20 (s, 1H, -NH), 6,95 (s, 1H, H-1), 6,92 (s, 1H, H-4), 5,67 (s, 1H, 17β-OH), 3,74 (s, 3H, 2-OCH₃), 3,56-3,50 (m, 1H, H-17α), 2,80-2,62 (m, 2H, H-6), 1,97 (s, 3H, -NCOCH₃), 0,89 (s, 1H, H-18), 0,28-0,20 (m, 2H, 14α,15α-CH₂).

### Beispiel 6

### 2-Ethoxy-17β-hydroxy-estra-1,3,5(10)-trien-3-yl-N-acetylsulfamat 6:

Nach allgemeiner Synthesevorschrift 1, 2 und 3 wurden aus 1,40 mmol 2-Ethoxy-17-oxo-estra-1,3,5(10)-trien-3-ol 0,94 mmol **8** (67 % d. Th.) erhalten.
¹H-NMR (400 MHz, DMSO-d₆, TMS) δ (ppm): 12,20 (s, 1H, -NH), 6,99 (s, 1H, H-1), 6,86 (s, 1H, H-4), 4,53 (d, J = 4,7 Hz, 1H, 17β-OH), 4,07-3,96 (m, 3H, 2-OCH₂CH₃), 3,56-3,50 (m, 1H, H-17α), 2,74-2,68 (m, 2H, H-6), 1,97 (s, 3H, -NCOCH₃), 1,29 (t, 3H, 2-OCH₂CH₃), 0,68 (s, 3H, H-18).

### Beispiel 7

### 2-Methoxy-17α-hydroxy-14α,15α-methylen-estra-1,3,5(10),8-tetraen-3-yl-sulfamat 7:

Nach allgemeiner Synthesevorschrift 1 und anschließender Abspaltung der *tert*-Butyldimethylsilyl-Schutzgruppe (T. W. Greene, P. G. M. Wuts, Protective Groups in Organic Synthesis, J. Wiley & Sons, 1999, S.273-276) wurden aus 1,67 mmol 17α-*tert*-Butyldimethylsilyloxy-2-methoxy-14α,15α-methylen-estra-1,3,5(10),8-tetraen-3-ol 1,08 mmol **11** (65 % d. Th.) erhalten.
¹H-NMR (400 MHz, DMSO-d₆, TMS) δ (ppm): 7,81 (s, 2H, -NH₂), 6,98 (s, 1H, H-1), 6,93 (s, 1 H, H-4), 3,90 (d, J = 6,2 Hz, 1 H, H-17β), 3,80 (s, 3H, 2-OCH₃), 2,80-2,62 (m, 2H, H-6), 1,28-1,25 (m, 1H, 14α,15α-CH₂), 0,93 (s, 1H, H-18), 0,47-0,43 (m, 1H, 14α,15α-CH₂).

### Beispiel 8

### 2-Methoxy-17α-hydroxy-14α,15α-methylen-estra-1,3,5(10),8-tetraen-3-yl-N-acetylsulfamat 8:

Nach allgemeiner Synthesevorschrift 1 und 2 sowie anschließender Abspaltung der *tert-*Butyldimethylsilyl-Schutzgruppe (T. W. Greene, P. G. M. Wuts, Protective Groups in Organic Synthesis, J. Wiley & Sons, 1999, S.273-276) wurden aus 1,89 mmol 17*α-tert-*Butyldimethylsilyloxy-2-methoxy-14α,15α-methylen-estra-1,3,5(10),8-tetraen-3-ol 0,91 mmol **12** (48 % d. Th.) erhalten.
¹H-NMR (400 MHz, DMSO-d₆, TMS) δ (ppm): 12,20 (s, 1H, -NH), 6,98 (s, 1H, H-1), 6,90 (s, 1H, H-4), 3,89 (d, J = 6,1 Hz, 1 H, H-17β), 3,80 (s, 3H, 2-OCH₃), 2,80-2,62 (m, 2H, H-6), 1,96 (s, 3H, -NCOCH₃), 1,28-1,24 (m, 1H, 14α,15α-CH₂), 0,93 (s, 1H, H-18), 0,47-0,40 (m, 1H, 14α,15α-CH₂).

## Patentansprüche

1. Verwendung von Verbindungen der allgemeinen Formel 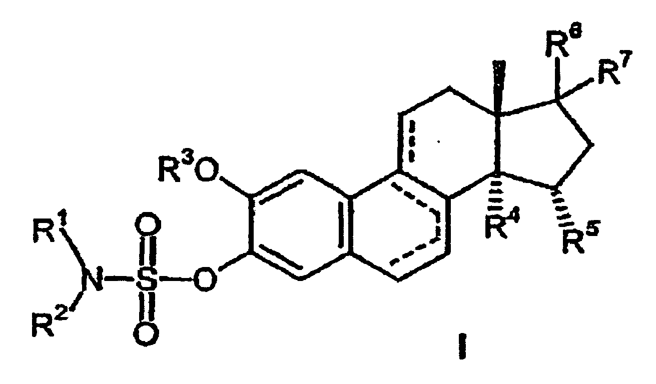 worin
R¹ und R² unabhängig voneinander H, Methyl, C₁-C₄-Acyl, Benzyl,
R³ C₁-C₄-Alkyl oder einen Rest der Formel CₙFₘHₒ, wobei n = 1, 2, 3, 4, 5 oder 6, m > 1 und m + o = 2n + 1 ist, bedeutet,
R⁴ und R⁵ jeweils H oder zusammen eine Methylengruppe bzw. eine zusätzliche Doppelbindung,
R⁶ H,
R⁷ OH, OC₁-C₄-Alkyl, OC₁-C₁₁Acyl oder OSO₂NR¹R² darstellen,
wobei im B- und C-Ring des Steroidgerüstes die gestrichelten Linien zusätzlich bis zu zwei Doppelbindungen sein können,
zur Herstellung von Arzneimitteln zur Behandlung von Tumorerkrankungen, die sich durch die Hemmung der Tubulinpolymerisation positiv beeinflussen lassen, mit der Maßgabe, daß die Verbindung etwas anderes ist als

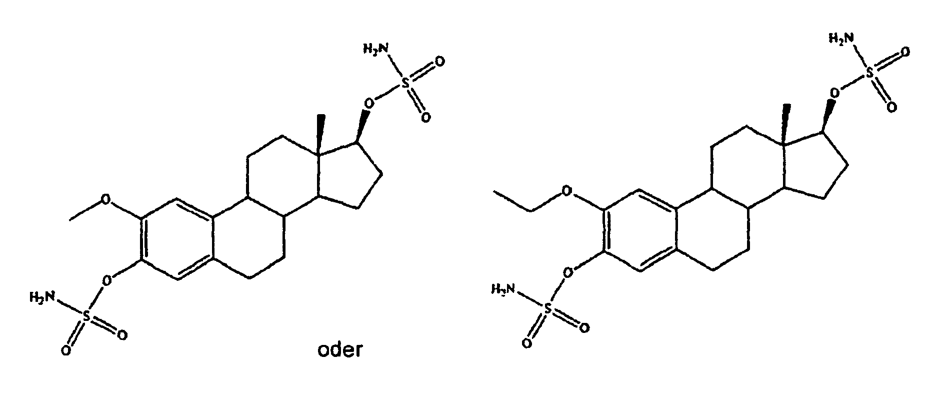

2. Verwendung nach Anspruch 1, **dadurch gekennzeichnet, daß** R³ Methyl Ethyl oder 2,2,2-Trifluorethyl darstellt.

3. Verwendung nach einem der Ansprüche 1 bis 2, **dadurch gekennzeichnet, daß** R¹ H bedeutet.

4. Verwendung nach einem der Ansprüche 1 bis 3, **dadurch gekennzeichnet, daß** R² H bedeutet.

5. Verwendung nach Anspruch 4, **dadurch gekennzeichnet, daß** R³ eine Methylgruppe darstellt.

6. Verwendung nach einem der vorherigen Ansprüche, nämlich
1) 2-Methoxy-17β-hydroxy-estra-1,3,5(10)-trien-3-yl-sulfamat
2) 2-Methoxy-17α-hydroxy-estra-1,3,5(10)-trien-3-yl-sulfamat
3) 2-Methoxy-17β-hydroxy-estra-1,3,5(10)-trien-3-yl-*N*-acetylsulfamat
4) 2-Methoxy-17a-hydroxy-estra-1,3,5(10)-trien-3-yl-*N*-acetylsulfamat
5) 2-Methoxy-17β-hydroxy-14α,15α-methylen-estra-1,3,5(10)-trien-3-yl-sulfamat
6) 2-Methoxy-17α-hydroxy-14α,15α-methylen-estra-1,3,5(10)-trien-3-yl-sulfamat
7) 2-Methoxy-17β-hydroxy-14α,15α-methylen-estra-1,3,5(10)-trien-3-yl-*N-*acetylsulfamat
8) 2-Methoxy-17α-hydroxy-14α,15α-methylen-estra-1,3,5(10)-trien-3-yl-*N-*acetylsulfamat
9) 2-Methoxy-17β-hydroxy-14α,15α-methylen-estra-1,3,5(10),8-tetraen-3-yl-sulfamat
10) 2-Methoxy-17α-hydroxy-14α,15α-methylen-estra-1,3,5(10),8-tetraen-3-yl-sulfamat
11) 2-Methoxy-17β-hydroxy-14α,15α-methylen-estra-1,3,5(10),8-tetraen-3-yl-sulfamat
12) 2-Methoxy-17α-hydroxy-14α,15α-methylen-estra-1,3,5(10),8-tetraen-3-yl-*N-*acetylsulfamat
13) 2-Ethoxy-17β-hydroxy-estra-1,3,5(10)-trien-3-yl-sulfamat
14) 2-Ethoxy-17α-hydroxy-estra-1,3,5(10)-trien-3-yl-sulfamat
15) 2-Ethoxy-17β-hydroxy-estra-1,3,5(10)-trien-3-yl-*N*-acetylsulfamat
16) 2-Ethoxy-17α-hydroxy-estra-1,3,5(10)-trien-3-yl-*N*-acetylsulfamat
17) 2-Ethoxy-17β-hydroxy-14α,15α-methylen-estra-1,3,5(10)-trien-3-yl-sulfamat
18) 2-Ethoxy-17α-hydroxy-14α,15α-methylen-estra-1,3,5(10)-trien-3-yl-sulfamat
19) 2-Ethoxy-17β-hydroxy-14α,15α-methylen-estra-1,3,5(10)-trien-3-yl-*N-*acetylsulfamat
20) 2-Ethoxy-17α-hydroxy-14α,15α-methylen-estra-1,3,5(10)-trien-3-yl-*N-*acetylsulfamat
21) 2-Ethoxy-17β-hydroxy-14α,15α-methylen-estra-1,3,5(10),8-tetraen-3-yl-sulfamat
22) 2-Ethoxy-17α-hydroxy-14α,15α-methylen-estra-1,3,5(10),8-tetraen-3-yl-sulfamat
23) 2-Ethoxy-17β-hydroxy-14α,15α-methylen-estra-1,3,5(10),8-tetraen-3-yl-*N-*acetylsulfamat
24) 2-Ethoxy-17α-hydroxy-14α,15α-methylen-estra-1,3,5(10),8-tetraen-3-yl-*N-*acetylsulfamat
25) 2-(2',2',2'-Trifluorethoxy)-17β-hydroxy-estra-1,3,5(10)-trien-3-yl-sulfamat
26) 2-(2',2',2'-Trifluorethoxy)-17α-hydroxy-estra-1,3,5(10)-trien-3-yl-sulfamat
27) 2-(2',2',2'-Trifluorethoxy)-17β-hydroxy-estra-1,3,5(10)-trien-3-yl-*N*-acetylsulfamat
28) 2-(2',2',2'-Trifluorethoxy)-17α-hydroxy-estra-1,3,5(10)-trien-3-yl-*N*-acetylsulfamat
29) 2-(2',2',2'-Trifluorethoxy)-17β-hydroxy-14α,15α-methylen-estra-1,3,5(10)-trien-3-yl-sulfamat
30) 2-(2',2',2'-Trifluorethoxy)-17α-hydroxy-14α,15α-methylen-estra-1,3,5(10)-trien-3-yl-sulfamat
31) 2-(2',2',2'-Trifluorethoxy)-17β-hydroxy-14α,15α-methylen-estra-1,3,5(10),8-tetraen-3-yl-sulfamat
32) 2-(2',2',2'-Trifluorethoxy)-17α-hydroxy-14α,15α-methylen-estra-1,3,5(10),8-tetraen-3-yl-sulfamat
33) 2-(2',2',2'-Trifluorethoxy)-17β-hydroxy-14α,15α-methylen-estra-1,3,5(10)-trien-3-yl-*N*-acetylsulfamat
34) 2-(2',2',2'-Trifluorethoxy)-17α-hydroxy-14α,15α-methylen-estra-1,3,5(10)-trien-3-yl-*N*-acetylsulfamat
35) 2-(2',2',2'-Trifluorethoxy)-17β-hydroxy-14α,15α-methylen-estra-1,3,5(10),8-tetraen-3-yl-*N*-acetylsulfamat
36) 2-(2',2',2'-Trifluorethoxy)-17α-hydroxy-14α,15α-methylen-estra-1,3,5(10),8-tetraen-3-yl-*N*-acetylsulfamat

7. Verwendung von Verbindungen nach einem der vorherigen Ansprüche zur Herstellung eines Arzneimittels zur Behandlung von Tumorerkrankungen der männlichen und weiblichen Keimdrüsen und der männlichen und weiblichen Sexualorgane einschließlich der Brustdrüsen.

8. Verwendung von Verbindungen nach einem der vorherigen Ansprüche zur Herstellung eines Arzneimittels zur Behandlung von Mammakarzinom.

9. Verwendung von Verbindungen nach einem der vorherigen Ansprüche zur Herstellung eines Arzneimittels zur Behandlung von Prostatakarzinom.

10. 2-Alkoxyestradiolsulfamate, nämlich
1) 2-Methoxy-17β-hydroxy-estra-1,3,5(10)-trien-3-yl-sulfamat
2) 2-Methoxy-17α-hydroxy-estra-1,3,5(10)-trien-3-yl-sulfamat
3) 2-Methoxy-17β-hydroxy-estra-1,3,5(10)-trien-3-yl-*N*-acetylsulfamat
4) 2-Methoxy-17α-hydroxy-estra-1,3,5(10)-trien-3-yl-*N*-acetylsulfamat
5) 2-Methoxy-17β-hydroxy-14α,15α-methylen-estra-1,3,5(10)-trien-3-yl-sulfamat
6) 2-Methoxy-17α-hydroxy-14α,15α-methylen-estra-1,3,5(10)-trien-3-yl-sulfamat
7) 2-Methoxy-17β-hydroxy-14α,15α-methylen-estra-1,3,5(10)-trien-3-yl-*N-*acetylsulfamat
8) 2-Methoxy-17α-hydroxy-14α,15α-methylen-estra-1,3,5(10)-trien-3-yl-*N-*acetylsulfamat
9) 2-Methoxy-17β-hydroxy-14α,15α-methylen-estra-1,3,5(10),8-tetraen-3-yl-sulfamat
10) 2-Methoxy-17α-hydroxy-14α,15α-methylen-estra-1,3,5(10),8-tetraen-3-yl-sulfamat
11) 2-Methoxy-17β-hydroxy-14α,15α-methylen-estra-1,3,5(10),8-tetraen-3-yl-sulfamat
12) 2-Methoxy-17α-hydroxy-14α,15α-methylen-estra-1,3,5(10),8-tetraen-3-yl-*N-*acetylsulfamat
13) 2-Ethoxy-17β-hydroxy-estra-1,3,5(10)-trien-3-yl-sulfamat
14) 2-Ethoxy-17α-hydroxy-estra-1,3,5(10)-trien-3-yl-sulfamat
15) 2-Ethoxy-17β-hydroxy-estra-1,3,5(10)-trien-3-yl-*N*-acetylsulfamat
16) 2-Ethoxy-17α-hydroxy-estra-1,3,5(10)-trien-3-yl-*N*-acetylsulfamat
17) 2-Ethoxy-17β-hydroxy-14α,15α-methylen-estra-1,3,5(10)-trien-3-yl-sulfamat
18) 2-Ethoxy-17α-hydroxy-14α,15α-methylen-estra-1,3,5(10)-trien-3-yl-sulfamat
19) 2-Ethoxy-17β-hydroxy-14α,15α-methylen-estra-1,3,5(10)-trien-3-yl-*N-*acetylsulfamat
20) 2-Ethoxy-17α-hydroxy-14α,15α-methylen-estra-1,3,5(10)-trien-3-yl-*N-*acetylsulfamat
21) 2-Ethoxy-17β-hydroxy-14α,15α-methylen-estra-1,3,5(10),8-tetraen-3-yl-sulfamat
22) 2-Ethoxy-17a-hydroxy-14a,15a-methylen-estra-1,3,5(10),8-tetraen-3-yl-sulfamat
23) 2-Ethoxy-17β-hydroxy-14α,15α-methylen-estra-1,3,5(10),8-tetraen-3-yl-*N-*acetylsulfaniat
24) 2-Ethoxy-17a-hydroxy-14a,15a-methylen-estra-1,3,5(10),8-tetraen-3-yl-*N-*acetylsulfamat
25) 2-(2',2',2'-Trifluorethoxy)-17β-hydroxy-estra-1,3,5(10)-trien-3-yl-sulfamat
26) 2-(2',2',2'-Trifluorethoxy)-17α-hydroxy-estra-1,3,5(10)-trien-3-yl-sulfamat
27) 2-(2',2',2'-Trifluorethoxy)-17β-hydroxy-estra-1,3,5(10)-trien-3-yl-*N*-acetylsulfamat
28) 2-(2',2',2'-Trifluorethoxy)-17a-hydroxy-estra-1,3,5(10)-trien-3-yl-*N*-acetylsulfamat
29) 2-(2',2',2'-Trifluorethoxy)-17β-hydroxy-14α,15α-methylen-estra-1,3,5(10)-trien-3-yl-sulfamat
30) 2-(2',2',2'-Trifluorethoxy)-17α-hydroxy-14α,15α-methylen-estra-1,3,5(10)-trien-3-yl-sulfamat
31) 2-(2',2',2'-Trifluorethoxy)-17β-hydroxy-14α,15α-methylen-estra-1,3,5(10),8-tetraen-3-yl-sulfamat
32) 2-(2',2',2'-Trifluorethoxy)-17α-hydroxy-14α,15α-methylen-estra-1,3,5(10),8-tetraen-3-yl-sulfamat
33) 2-(2',2',2'-Trifluorethoxy)-17β-hydroxy-14α,15α-methylen-estra-1,3,5(10)-trien-3-yl-*N*-acetylsulfamat
34) 2-(2',2',2'-Trifluorethoxy)-17α-hydroxy-14α,15α-methylen-estra-1,3,5(10)-trien-3-yl-*N*-acetylsulfamat
35) 2-(2',2',2'-Trifluorethoxy)-17β-hydroxy-14α,15α-methylen-estra-1,3,5(10),8-tetraen-3-yl-*N*-acetylsulfamat
36) 2-(2',2',2'-Trifluorethoxy)-17α-hydroxy-14α,15α-methylen-estra-1,3,5(10),8-tetraen-3-yl-*N*-acetylsulfamat

11. Pharmazeutische Zusammensetzungen, enthaltend mindestens eine Verbindung nach Anspruch 10 zusammen mit pharmazeutisch verträglichen Hilfs- und/oder Trägerstoffen.

## Claims

1. Use of compounds with general formula: 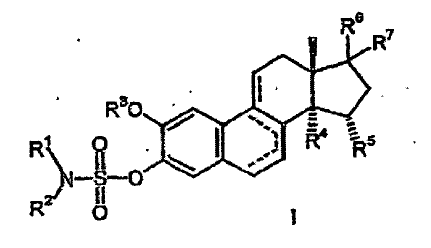 in which:
R¹ and R² independently represent H, methyl, C₁-C₄-acyl, benzyl;
R³ represents C₁-C₄ alkyl or a residue with formula CₙFₘHₒ, where n=1, 2, 3, 4, 5 or 6, m > 1 and m+o=2n+1;
R⁴ and R⁵ each represent H or together represent a methylene group or an additional double bond;
R⁶ represents H;
R⁷ represents OH, OC₁-C₄-alkyl, OC₁-C₁₁-acyl or OSO₂NR¹R²;
wherein in the B- and C-ring of the steroid framework, the dotted lines can also be up to two double bonds;
for the production of drugs for the treatment of tumoral diseases which are positively influenced by inhibiting tubulin polymerization
with the proviso that the compound is other than

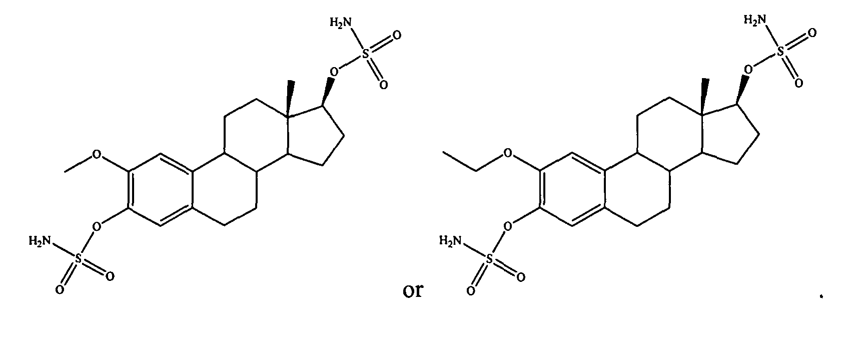

2. Use according to claim 1, **characterized in that** R³ represents methyl, ethyl or 2,2,2-trifluoroethyl.

3. Use according to claim 1 or claim 2, **characterized in that** R¹ represents H.

4. Use according to one of claims 1 to 3, **characterized in that** R² represents H.

5. Use according to claim 4, **characterized in that** R³ represents a methyl group.

6. Use according to one of the preceding claims, namely of:
1) 2-methoxy-17β-hydroxy-estra-1,3,5(10)-triene-3-yl-sulphamate;
2) 2-methoxy-17α-hydroxy-estra-1,3,5(10)-triene-3-yl-sulphamate;
3) 2-methoxy-17β-hydroxy-estra-1,3,5(10)-triene-3-yl-N-acetylsulphamate;
4) 2-methoxy-17α-hydroxy-estra-1,3,5(10)-triene-3-yl-N-acetylsulphamate;
5) 2-methoxy-17β-hydroxy-14α,15α-methylene-estra-1,3,5(10)-triene-3-yl-sulphamate;
6) 2-methoxy-17α-hydroxy-14α,15α-methylene-estra-1,3,5(10)-triene-3-yl-sulphamate;
7) 2-methoxy-17β-hydroxy-14α,15α-methylene-estra-1,3,5(10)-triene-3-yl-N-acetylsulphamate;
8) 2-methoxy-17α-hydroxy-14α,15α-methylene-estra-1,3,5(10)-triene-3-yl-N-acetylsulphamate;
9) 2-methoxy-17β-hydroxy-14α,15α-methylene-estra-1,3,5(10),8-tetraene-3-yl-sulphamate;
10) 2-methoxy-17α-hydroxy-14α,15α-methylene-estra-1,3,5(10),8-tetraene-3-yl-sulphamate;
11) 2-methoxy-17β-hydroxy-14α,15α-methylene-estra-1,3,5(10),8-tetraene-3-yl-N-acetylsulphamate;
12) 2-methoxy-17α-hydroxy-14α,15α-methylene-estra-1,3,5(10),8-tetraene-3-yl-N-acetylsulphamate;
13) 2-ethoxy-17β-hydroxy-estra-1,3,5(10)-triene-3-yl-sulphamate;
14) 2-ethoxy-17α-hydroxy-estra-1,3,5(10)-triene-3-yl-sulphamate;
15) 2-ethoxy-17β-hydroxy-estra-1,3,5(10)-triene-3-yl-N-acetylsulphamate;
16) 2-ethoxy-17α-hydroxy-estra-1,3,5(10)-triene-3-yl-N-acetylsulphamate;
17) 2-ethoxy-17β-hydroxy-14α,15α-methylene-estra-1,3,5(10)-triene-3-yl-sulphamate;
18) 2-ethoxy-17α-hydroxy-14α,15α-methylene-estra-1,3,5(10)-triene-3-yl-sulphamate;
19) 2-ethoxy-17β-hydroxy-14α,15α-methylene-estra-1,3,5(10)-triene-3-yl-N-acetylsulphamate;
20) 2-ethoxy-17α-hydroxy-14α,15α-methylene-estra-1,3,5(10)-triene-3-yl-N-acetylsulphamate;
21) 2-ethoxy-17β-hydroxy-14α,15α-methylene-estra-1,3,5(10)-8-tetraene-3-yl-sulphamate;
22) 2-ethoxy-17α-hydroxy-14α,15α-methylene-estra-1,3,5(10)-8-tetraene-3-yl-sulphamate;
23) 2-ethoxy-17β-hydroxy-14α,15α-methylene-estra-1,3,5(10),8-tetraene-3-yl-N-acetylsulphamate;
24) 2-ethoxy-17α-hydroxy-14α,15α-methylene-estra-1,3,5(10)-8-tetraene-3-yl-N-acetylsulphamate;
25) 2-(2',2',2'-trifluoroethoxy)-17β-hydroxy-estra-1,3,5(10)-triene-3-yl-sulphamate;
26) 2-(2',2',2'-trifluoroethoxy)-17α-hydroxy-estra-1,3,5(10)-triene-3-yl-sulphamate;
27) 2-(2',2',2'-trifluoroethoxy)-17β-hydroxy-estra-1,3,5(10)-triene-3-yl-N-acetylsulphamate;
28) 2-(2',2',2'-trifluoroethoxy)-17α-hydroxy-estra-1,3,5(10)-triene-3-yl-N-acetylsulphamate;
29) 2-(2',2',2'-trifluoroethoxy)-17β-hydroxy-14α,15α-methylene-estra-1,3,5(10)-triene-3-yl-sulphamate;
30) 2-(2',2',2'-trifluoroethoxy)-17α-hydroxy-14α,15α-methylene-estra-1,3,5(10)-triene-3-yl-sulphamate;
31) 2-(2',2',2'-trifluoroethoxy)-17β-hydroxy-14α,15α-methylene-estra-1,3,5(10),8-tetraene-3-yl-sulphamate;
32) 2-(2',2',2'-trifluoroethoxy)-17α-hydroxy-14α,15α-methylene-estra-1,3,5(10),8-tetraene-3-yl-sulphamate;
33) 2-(2',2',2'-trifluoroethoxy)-17β-hydroxy-14α,15α-methylene-estra-1,3,5(10)-triene-3-yl-N-acetylsulphamate;
34) 2-(2',2',2'-trifluoroethoxy)-17α-hydroxy-14α,15α-methylene-estra-1,3,5(10)-triene-3-yl-N-acetylsulphamate;
35) 2-(2',2',2'-trifluoroethoxy)-17β-hydroxy-14α,15α-methylene-estra-1,3,5(10),8-tetraene-3-yl-N-acetylsulphamate;
36) 2-(2',2',2'-trifluoroethoxy)-17α-hydroxy-14α,15α-methylene-estra-1,3,5(10),8-tetraene-3-yl-N-acetylsulphamate.

7. Use of compounds according to one of the preceding claims, for the production of a drug for the treatment of tumoral diseases of the male and female reproductive glands and the male and female sexual organs including the mammary glands.

8. Use of compounds according to one of the preceding claims, for the production of a drug for the treatment of breast cancer.

9. Use of compounds according to one of the preceding claims, for the production of a drug for the treatment of prostate cancer.

10. 2-alkoxyoestradiosulphamates, namely:
1) 2-methoxy-17β-hydroxy-estra-1,3,5(10)-triene-3-yl-sulphamate;
2) 2-methoxy-17α-hydroxy-estra-1,3,5(10)-triene-3-yl-sulphamate;
3) 2-methoxy-17β-hydroxy-estra-1,3,5(10)-triene-3-yl-N-acetylsulphamate;
4) 2-methoxy-17α-hydroxy-estra-1,3,5(10)-triene-3-yl-N-acetylsulphamate;
5) 2-methoxy-17β-hydroxy-14α,15α-methylene-estra-1,3,5(10)-triene-3-yl-sulphamate;
6) 2-methoxy-17α-hydroxy-14α,15α-methylene-estra-1,3,5(10)-triene-3-yl-sulphamate;
7) 2-methoxy-17β-hydroxy-14α,15α-methylene-estra-1,3,5(10)-triene-3-yl-N-acetylsulphamate;
8) 2-methoxy-17α-hydroxy-14α,15α-methylene-estra-1,3,5(10)-triene-3-yl-N-acetylsulphamate;
9) 2-methoxy-17β-hydroxy-14α,15α-methylene-estra-1,3,5(10),8-tetraene-3-yl-sulphamate;
10) 2-methoxy-17α-hydroxy-14α,15α-methylene-estra-1,3,5(10),8-tetraene-3-yl-sulphamate;
11) 2-methoxy-17β-hydroxy-14α,15α-methylene-estra-1,3,5(10),8-tetraene-3-yl-N-acetylsulphamate;
12) 2-methoxy-17α-hydroxy-14α,15α-methylene-estra-1,3,5(10),8-tetraene-3-yl-N-acetylsulphamate;
13) 2-ethoxy-17β-hydroxy-estra-1,3,5(10)-triene-3-yl-sulphamate;
14) 2-ethoxy-17α-hydroxy-estra-1,3,5(10)-triene-3-yl-sulphamate;
15) 2-ethoxy-17β-hydroxy-estra-1,3,5(10)-triene-3-yl-N-acetylsulphamate;
16) 2-ethoxy-17α-hydroxy-estra-1,3,5(10)-triene-3-yl-N-acetylsulphamate;
17) 2-ethoxy-17β-hydroxy-14α,15α-methylene-estra-1,3,5(10)-triene-3-yl-sulphamate;
18) 2-ethoxy-17α-hydroxy-14α,15α-methylene-estra-1,3,5(10)-triene-3-yl-sulphamate;
19) 2-ethoxy-17β-hydroxy-14α,15α-methylene-estra-1,3,5(10)-triene-3-yl-N-acetylsulphamate;
20) 2-ethoxy-17α-hydroxy-14α,15α-methylene-estra-1,3,5(10)-triene-3-yl-N-acetylsulphamate;
21) 2-ethoxy-17β-hydroxy-14α,15α-methylene-estra-1,3,5(10),8-tetraene-3-yl-sulphamate;
22) 2-ethoxy-17α-hydroxy-14α,15α-methylene-estra-1,3,5(10),8-tetraene-3-yl-sulphamate;
23) 2-ethoxy-17β-hydroxy-14α,15α-methylene-estra-1,3,5(10),8-tetraene-3-yl-N-acetylsulphamate;
24) 2-ethoxy-17α-hydroxy-14α,15α-methylene-estra-1,3,5(10),8-tetraene-3-yl-N-acetylsulphamate;
25) 2-(2',2',2'-trifluoroethoxy)-17β-hydroxy-estra-1,3,5(10)-triene-3-yl-sulphamate;
26) 2-(2',2',2'-trifluoroethoxy)-17α-hydroxy-estra-1,3,5(10)-triene-3-yl-sulphamate;
27) 2-(2',2',2'-trifluoroethoxy)-17β-hydroxy-estra-1,3,5(10)-triene-3-yl-N-acetylsulphamate;
28) 2-(2',2',2'-trifluoroethoxy)-17α-hydroxy-estra-1,3,5(10)-triene-3-yl-N-acetylsulphamate;
29) 2-(2',2',2'-trifluoroethoxy)-17β-hydroxy-14α,15α-methylene-estra-1,3,5(10)-triene-3-yl-sulphamate;
30) 2-(2',2',2'-trifluoroethoxy)-17α-hydroxy-14α,15α-methylene-estra-1,3,5(10)-triene-3-yl-sulphamate;
31) 2-(2',2',2'-trifluoroethoxy)-17β-hydroxy-14α,15α-methylene-estra-1,3,5(10),8-tetraene-3-yl-sulphamate;
32) 2-(2',2',2'-trifluoroethoxy)-17α-hydroxy-14α,15α-methylene-estra-1,3,5(10),8-tetraene-3-yl-sulphamate;
33) 2-(2',2',2'-trifluoroethoxy)-17β-hydroxy-14α,15α-methylene-estra-1,3,5(10)-triene-3-yl-N-acetylsulphamate;
34) 2-(2',2',2'-trifluoroethoxy)-17α-hydroxy-14α,15α-methylene-estra-1,3,5(10)-triene-3-yl-N-acetylsulphamate;
35) 2-(2',2',2'-trifluoroethoxy)-17β-hydroxy-14α,15α-methylene-estra-1,3,5(10),8-tetraene-3-yl-N-acetylsulphamate;
36) 2-(2',2',2'-trifluoroethoxy)-17α-hydroxy-14α,15α-methylene-estra 1,3,5(10),8-tetraene-3-yl-N-acetylsulphamate.

11. Pharmaceutical compositions comprising at least one compound according to claim 10, together with pharmaceutically acceptable excipients and/or vehicles.

## Revendications

1. Utilisation de composés de formule générale 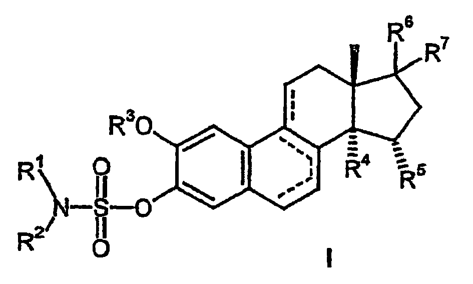 dans laquelle
R¹ et R² représentent indépendamment l'un de l'autre H, un reste méthyle, acyle en C₁ à C₄, benzyle,
R³ représente un reste alkyle en C₁ à C₄ ou un reste de formule CₙFₘHₒ dans laquelle n a la valeur 1, 2, 3, 4, 5 ou 6, m est supérieur à 1 et la somme m + o est égale à 2n + 1,
R⁴ et R⁵ représentent chacun H ou forment ensemble un groupe méthylène ou une double liaison complémentaire,
R⁶ représente H,
R⁷ représente OH, un reste O(alkyle en C₁ à C₄), O(acyle en C₁ à C₁₁) ou OSO₂NR¹R²,
les segments en traits interrompus dans les noyaux B et C du squelette stéroïde pouvant représenter en outre jusqu'à deux doubles liaisons,
pour la préparation d'un médicament destiné au traitement de maladies tumorales qui peuvent être influencées positivement par l'inhibition de la polymérisation de tubuline,
sous réserve que le composé soit autre que

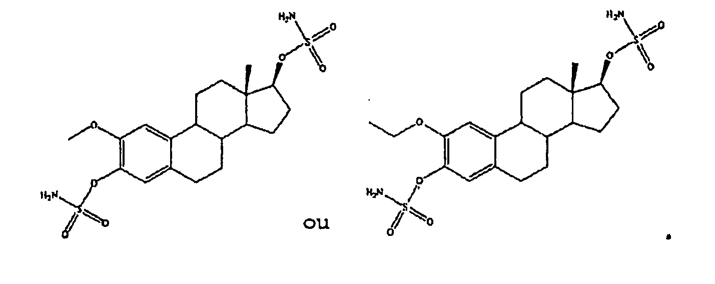

2. Utilisation suivant la revendication 1, **caractérisée en ce que** R³ est un reste méthyle, éthyle ou 2,2,2-trifluoréthyle.

3. Utilisation suivant l'une des revendications 1 à 2, **caractérisée en ce que** R¹ représente H.

4. Utilisation suivant l'une des revendications 1 à 3, **caractérisée en ce que** R² représente H.

5. Utilisation suivant la revendication 4, **caractérisée en ce que** R³ représente un groupe méthyle.

6. Utilisation suivant l'une des revendications précédentes, à savoir :
1) 2-méthoxy-17β-hydroxy-estra-1,3,5(10)-trién-3-yl-sulfamate
2) 2-méthoxy-17α-hydroxy-estra-1,3,5(10)-trién-3-yl-sulfamate
3) 2-méthoxy-17β-hydroxy-estra-1,3,5(10)-trién-3-yl-*N*-acétylsulfamate
4) 2-méthoxy-17α-hydroxy-estra-1,3,5(10)-trién-3-yl-*N*-acétylsulfamate
5) 2-méthoxy-17β-hydroxy-14α,15α-méthylène-estra-1,3,5(10)-trién-3-yl-sulfamate
6) 2-méthoxy-17α-hydroxy-14α,15α-méthylène-estra-1,3,5(10)-trién-3-yl-sulfamate
7) 2-méthoxy-17β-hydroxy-14α,15α-méthylène-estra-1,3,5(10)-trién-3-yl-*N*-acétylsulfamate
8) 2-méthoxy-17α-hydroxy-14α,15α-méthylène-estra-1,3,5(10)-trién-3-yl-*N*-acétylsulfamate
9) 2-méthoxy-17β-hydroxy-14α,15α-méthylène-estra-1,3,5(10),8-tétraén-3-yl-sulfamate
10) 2-méthoxy-17α-hydroxy-14α,15α-méthylène-estra-1,3,5(10),8-tétraén-3-yl-sulfamate
11) 2-méthoxy-17β-hydroxy-14α,15α-méthylène-estra-1,3,5(10),8-tétraén-3-yl-*N*-acétylsulfamate
12) 2-méthoxy-17α-hydroxy-14α,15α-méthylène-estra-1,3,5(10),8-tétraén-3-yl-*N*-acétylsulfamate
13) 2-éthoxy-17β-hydroxy-estra-1,3,5(10)-trién-3-yl-sulfamate
14) 2-éthoxy-17α-hydroxy-estra-1,3,5(10)-trién-3-yl-sulfamate
15) 2-éthoxy-17β-hydroxy-estra-1,3,5(10)-trién-3-yl-*N*-acétylsulfamate
16) 2-éthoxy-17α-hydroxy-estra-1,3,5(10)-trién-3-yl-*N*-acétylsulfamate
17) 2-éthoxy-17β-hydroxy-14α,15α-méthylène-estra-1,3,5(10)-trién-3-yl-sulfamate
18) 2-éthoxy-17α-hydroxy-14α,15α-méthylène-estra-1,3,5(10)-trién-3-yl-sulfamate
19) 2-éthoxy-17β-hydroxy-14α,15α-méthylène-estra-1,3,5(10)-trién-3-yl-*N*-acétylsulfamate
20) 2-éthoxy-17α-hydroxy-14α,15α-méthylène-estra-1,3,5(10)-trién-3-yl-*N*-acétylsulfamate
21) 2-éthoxy-17β-hydroxy-14α,15α-méthylène-estra-1,3,5(10),8-tétraén-3-yl-sulfamate
22) 2-éthoxy-17α-hydroxy-14α,15α-méthylène-estra-1,3,5(10),8-tétraén-3-yl-sulfamate
23) 2-éthoxy-17β-hydroxy-14α,15α-méthylène-estra-1,3,5(10),8-tétraén-3-yl-*N*-acétylsulfamate
24) 2-éthoxy-17α-hydroxy-14α,15α-méthylène-estra-1,3,5(10),8-tétraén-3-yl-*N*-acétylsulfamate
25) 2-(2',2',2'-trifluoréthoxy)-17β-hydroxy-estra-1,3,5(10)-trién-3-yl-sulfamate
26) 2-(2',2',2'-trifluoréthoxy)-17α-hydroxy-estra-1,3,5(10)-trién-3-yl-sulfamate
27) 2-(2',2',2'-trifluoréthoxy)-17β-hydroxy-estra-1,3,5(10)-trién-3-yl-*N*-acétylsulfamate
28) 2-(2',2',2'-trifluoréthoxy)-17α-hydroxy-estra-1,3,5(10)-trién-3-yl-*N*-acétylsulfamate
29) 2-(2',2',2'-trifluoréthoxy)-17β-hydroxy-14α,15α-méthylène-estra-1,3,5(10)-trién-3-yl-sulfamate
30) 2-(2',2',2'-trifluoréthoxy)-17α-hydroxy-14α,15α-méthylène-estra-1,3,5(10)-trién-3-yl-sulfamate
31) 2-(2',2',2'-trifluoréthoxy)-17β-hydroxy-14α,15α-méthylène-estra-1,3,5(10),8-tétraén-3-yl-sulfamate
32) 2-(2',2',2'-trifluoréthoxy)-17α-hydroxy-14α,15α-méthylène-estra-1,3,5(10),8-tétraén-3-yl-sulfamate
33) 2-(2',2',2'-trifluoréthoxy)-17β-hydroxy-14α,15α-méthylène-estra-1,3,5(10)-trién-3-yl-*N*-acétylsulfamate
34) 2-(2',2',2'-trifluoréthoxy)-17α-hydroxy-14α,15α-méthylène-estra-1,3,5(10)-trién-3-yl-*N*-acétylsulfamate
35) 2-(2',2',2'-trifluoréthoxy)-17β-hydroxy-14α,15α-méthylène-estra-1,3,5(10),8-tétraén-3-yl-*N*-acétylsulfamate
36) 2-(2',2',2'-trifluoréthoxy)-17α-hydroxy-14α,15α-méthylène-estra-1,3,5(10),8-tétraén-3-yl-*N*-acétylsulfamate

7. Utilisation de composés suivant l'une des revendications précédentes, pour la préparation d'un médicament destiné au traitement de maladies tumorales des glandes génitales masculines et féminines, des organes sexuels masculins et féminins, y compris les glandes mammaires.

8. Utilisation de composés suivant l'une des revendications précédentes, pour la préparation d'un médicament destiné au traitement du cancer du sein.

9. Utilisation de composés suivant l'une des revendications précédentes, pour la préparation d'un médicament destiné au traitement de cancers de la prostate.

10. Sulfamates de 2-alkoxyestradiol, à savoir :
1) 2-méthoxy-17β-hydroxy-estra-1,3,5(10)-trién-3-yl-sulfamate
2) 2-méthoxy-17α-hydroxy-estra-1,3,5(10)-trién-3-yl-sulfamate
3) 2-méthoxy-17β-hydroxy-estra-1,3,5(10)-trién-3-yl-*N*-acétylsulfamate
4) 2-méthoxy-17α-hydroxy-estra-1,3,5(10)-trién-3-yl-*N*-acétylsulfamate
5) 2-méthoxy-17β-hydroxy-14α,15α-méthylène-estra-1,3,5(10)-trién-3-yl-sulfamate
6) 2-méthoxy-17α-hydroxy-14α,15α-méthylène-estra-1,3,5(10)-trién-3-yl-sulfamate
7) 2-méthoxy-17β-hydroxy-14α,15α-méthylène-estra-1,3,5(10)-trién-3-yl-*N*-acétylsulfamate
8) 2-méthoxy-17α-hydroxy-14α,15α-méthylène-estra-1,3,5(10)-trién-3-yl-*N*-acétylsulfamate
9) 2-méthoxy-17β-hydroxy-14α,15α-méthylène-estra-1,3,5(10),8-tétraén-3-yl-sulfamate
10) 2-méthoxy-17α-hydroxy-14α,15α-méthylène-estra-1,3,5(10),8-tétraén-3-yl-sulfamate
11) 2-méthoxy-17β-hydroxy-14α,15α-méthylène-estra-1,3,5(10),8-tétraén-3-yl-*N*-acétylsulfamate
12) 2-méthoxy-17α-hydroxy-14α,15α-méthylène-estra-1,3,5(10),8-tétraén-3-yl-*N*-acétylsulfamate
13) 2-éthoxy-17β-hydroxy-estra-1,3,5(10)-trién-3-yl-sulfamate
14) 2-éthoxy-17α-hydroxy-estra-1,3,5(10)-trién-3-yl-sulfamate
15) 2-éthoxy-17β-hydroxy-estra-1,3,5(10)-trién-3-yl-*N*-acétylsulfamate
16) 2-éthoxy-17α-hydroxy-estra-1,3,5(10)-trién-3-yl-*N*-acétylsulfamate
17) 2-éthoxy-17β-hydroxy-14α,15α-méthylène-estra-1,3,5(10)-trién-3-yl-sulfamate
18) 2-éthoxy-17α-hydroxy-14α,15α-méthylène-estra-1,3,5(10)-trién-3-yl-sulfamate
19) 2-éthoxy-17β-hydroxy-14α,15α-méthylène-estra-1,3,5(10)-trién-3-yl-*N*-acétylsulfamate
20) 2-éthoxy-17α-hydroxy-14α,15α-méthylène-estra-1,3,5(10)-trién-3-yl-*N*-acétylsulfamate
21) 2-éthoxy-17β-hydroxy-14α,15α-méthylène-estra-1,3,5(10),8-tétraén-3-yl-sulfamate
22) 2-éthoxy-17α-hydroxy-14α,15α-méthylène-estra-1,3,5(10),8-tétraén-3-yl-sulfamate
23) 2-éthoxy-17β-hydroxy-14α,15α-méthylène-estra-1,3,5(10),8-tétraén-3-yl-*N*-acétylsulfamate
24) 2-éthoxy-17α-hydroxy-14α,15α-méthylène-estra-1,3,5(10),8-tétraén-3-yl-*N*-acétylsulfamate
25) 2-(2',2',2'-trifluoréthoxy)-17β-hydroxy-estra-1,3,5(10)-trién-3-yl-sulfamate
26) 2-(2',2',2'-trifluoréthoxy)-17α-hydroxy-estra-1,3,5(10)-trién-3-yl-sulfamate
27) 2-(2',2',2'-trifluoréthoxy)-17β-hydroxy-estra-1,3,5(10)-trién-3-yl-*N*-acétylsulfamate
28) 2-(2',2',2'-trifluoréthoxy)-17α-hydroxy-estra-1,3,5(10)-trién-3-yl-*N*-acétylsulfamate
29) 2-(2',2',2'-trifluoréthoxy)-17β-hydroxy-14α,15α-méthylène-estra-1,3,5(10)-trién-3-yl-sulfamate
30) 2-(2',2',2'-trifluoréthoxy)-17α-hydroxy-14α,15α-méthylène-estra-1,3,5(10)-trién-3-yl-sulfamate
31) 2-(2',2',2'-trifluoréthoxy)-17β-hydroxy-14α,15α-méthylène-estra-1,3,5(10),8-tétraén-3-yl-sulfamate
32) 2-(2',2',2'-trifluoréthoxy)-17α-hydroxy-14α,15α-méthylène-estra-1,3,5(10),8-tétraén-3-yl-sulfamate
33) 2-(2',2',2'-trifluoréthoxy)-17β-hydroxy-14α,15α-méthylène-estra-1,3,5(10)-trién-3-yl-*N*-acétylsulfamate
34) 2-(2',2',2'-trifluoréthoxy)-17α-hydroxy-14α,15α-méthylène-estra-1,3,5(10)-trién-3-yl-*N*-acétylsulfamate
35) 2-(2',2',2'-trifluoréthoxy)-17β-hydroxy-14α,15α-méthylène-estra-1,3,5(10),8-tétraén-3-yl-*N*-acétylsulfamate
36) 2-(2',2',2'-trifluoréthoxy)-17α-hydroxy-14α,15α-méthylène-estra-1,3,5(10),8-tétraén-3-yl-*N*-acétylsulfamate.

11. Compositions pharmaceutiques contenant au moins un composé suivant la revendication 10, conjointement avec des substances auxiliaires et/ou des supports pharmaceutiquement acceptables.
